# EUROPEAN PATENT APPLICATION

(11) **EP 0 550 949 A1**
(43) Date of publication of application: **14.07.1993**
(21) Application number: 92306189.9
(22) Date of filing: 06.07.1992
(51) Int. Cl.: A61M 25/06, A61M 5/32

(54) **Shield assembly for catheter placement units and other needle-bearing devices**

(30) Priority: 09.01.1992 US 817998
(71) Applicant: UNIVERSITY OF FLORIDA, Gainesville, Florida 32611-2037 (US)
(72) Inventor: Melker, Richard J., Gainesville, Florida 32610 (US); Miller, Gary J., Gainesville, Florida 32601 (US)
(74) Representative: Allman, Peter John

(57) **Abstract**

A shield assembly to substantially eliminate needle stick injuries by contaminated needles of needle-bearing medical devices. The inventive shield assembly includes a needle assembly (10) having a needle cannula (12) and a hub (14), and a shield (20) movably mounted over the needle cannula and hub of the needle assembly and including a locking member (22) to retain the shield in a first position wherein the distal end of the needle cannula is exposed or a second position wherein the distal end of the needle cannula is recessed within the shield. In one embodiment of the invention, the inventive shield assembly further includes a catheter unit (32) slidably mounted over the shield.

## Description

### BACKGROUND OF THE INVENTION

### 1. Field of the Invention

This invention relates to safety devices for preventing needle stick injuries to individuals who handle needle-bearing medical devices such as hypodermic syringes and catheter placement units. More particularly, the invention relates to devices which shield the needle following use of the needle.

### 2. Description of Related Art

Accidental needle sticks with contaminated needles present a major health problem to hospital and medical facility patients and personnel. It is estimated that there are 800,000 needle sticks per year in the United States resulting in a cost of testing and care of approximately $500,000,000. While needle sticks have been a problem since the invention of hypodermic needles, the recognition of the transfer of the AIDS virus by needle sticks has amplified concern over this problem. A number of other viral and bacterial infections (such as Hepatitis B, tuberculosis, and malaria) can also be transmitted by accidental needle stick injuries.

Unprotected needles present a high risk to clean-up personnel and others who may come into contact with the contaminated needle after the used needle-bearing device has been set aside or discarded. To reduce this risk, some frequently used needle-bearing medical devices (such as hypodermic syringes) are typically equipped with caps placed over the needle. The cap is removed prior to use of the needle. Following the use of the needle, the cap is replaced on the needle. While much research has been directed towards developing safe needle caps, needle stick injuries are still common. The recapping of the needle requires dexterity and care on the part of the user. Additionally, the caps may separate from the needle after the needle has been discarded. Therefore, even those needle-bearing medical devices provided with caps do not satisfactorily reduce the occurrence of needle stick injuries. To address this problem, the Center for Disease Control recommends that needles not be recapped, but rather be discarded in special containers. However, these containers are not always available, especially in emergency situations.

One frequently utilized procedure which presents a high risk of needle stick injury is the placement of intravenous catheters. It is common practice in hospitals to maintain intravenous access to patients for repeated administration of therapeutic fluids or drawing of blood. While this practice avoids the discomfort and risk a patient would experience from additional needle sticks required to directly access the patient's circulatory system, the safe placement of catheters requires a substantial amount of skill.

Intravenous access is most frequently maintained using plastic catheters. Typically, such catheters are provided with a hollow beveled placement needle. In one frequently utilized catheter placement unit, the plastic catheter is positioned over a metal needle with only the tip of the needle protruding. To place such "over-the-needle catheters", medical personnel pierce a desired blood vessel with the needle tip and insert the needle and catheter therein. Once inserted, the catheter is advanced over the needle and the needle is withdrawn. The catheter may then be stabilized in place (e.g., by attaching its hub to the patient's skin with adhesive strips) and attached to an intravenous tubing system or syringe as desired.

Unfortunately, when the metal needle of standard over-the-needle catheter units is withdrawn from the catheter and the blood vessel, it becomes a source for potential needle stick injuries just like the contaminated needles of used hypodermic syringes.

To address the problem of needle stick injuries associated with catheter placement, a number of catheter placement units have been designed to include a needle-shielding mechanism. Such needle-shielding designs are disclosed for example in U.S. Patent Nos. 4,762,516 to Luther et al., 4,850,961 to Wanderer et al., 4,909,795 to Vining et al., and 4,417,887 to Koshi.

The needle-shielding design disclosed in each of the above mentioned patents, however, requires the user of the respective catheter placement unit to utilize a placement procedure which differs from and is more difficult than the procedure used to place standard over-the-needle catheters. Specifically, these designs isolate the user's hands from the tip of the catheter and thus increase the difficulty in guiding the catheter to its desired position. Additionally, the disclosed needle-shielding designs are adapted for use only with specific catheter placement units and thus do not decrease the risks of needle sticks associated with other needle-bearing medical devices.

A need continues to exist for a low cost, easy to use shield assembly which can be used with over-the-needle catheter placement assemblies as well as other needle-bearing devices such as hypodermic syringes.

### SUMMARY OF THE INVENTION

The present invention provides a shield assembly which substantially eliminates the risk of needle sticks by contaminated needles. More specifically, the present invention provides a shield assembly which covers and shields the used needle cannulas of hypodermic needles, catheter placement units and other needle-bearing medical devices which include needle assemblies provided with a hub.

The shield assembly of the present invention includes a needle assembly having a hollow needle cannula provided with a sharp distal end, and a hub connecting to the proximal end of the needle cannula. The inventive shield assembly further includes a shield movably mounted over the needle cannula and the hub of the needle assembly. The shield includes a locking member which cooperates with the hub of the needle assembly to retain the shield in a first position wherein the sharp distal end of the needle cannula is exposed or in a second position wherein the distal end of the needle cannula is recessed within, and thus covered by, the shield affording protection from the sharp distal end.

In one embodiment of the invention, the hub of the needle assembly is provided with a pair of grooves and the locking member of the shield assembly comprises a flexible clip adapted to seat into the grooves. When the shield assembly is positioned to place the flexible clip into the first, shallower groove of the pair of grooves, the distal end of the needle cannula is exposed. However, when the shield is slid forward to permit the flexible clip to seat into the deeper second groove, the distal end of the needle cannula is covered.

The clip preferably locks into the second groove so as not to be returnable to the first groove.

In another embodiment of the invention, the inventive shield assembly further includes a catheter which is slidably positioned over the shield. This embodiment permits placement of a catheter utilizing the standard over-the-needle catheter placement technique while preventing the occurrence of needle-stick injuries upon withdrawal of the needle.

The inventive shield assembly offers a number of advantages over prior art designs. Most importantly, it does not substantially interfere with the normal needle hub design with which users are familiar. Thus, use of the inventive shield assembly does not require acquisition of new psychomotor skills. Another advantageous feature of the present invention lies in its adaptability for use with any needle-bearing medical device which includes a needle assembly having a hub. The simple design of the device not only results in ease of use but also permits low cost manufacturing. The risk to medical and clean-up personnel of needle sticks by contaminated needles may thus be substantially eliminated in a cost-effective manner.

### BRIEF DESCRIPTION OF THE DRAWINGS

FIGURE 1 is a side cross-sectional view of the preferred embodiment of the invention showing the distal end of the needle cannula safely recessed within the shield.

FIGURE 2 is a side cross-sectional view of the embodiment of the invention depicted in FIGURE 1 with the distal end of the needle cannula exposed for insertion into a desired blood vessel.

FIGURE 3 is a perspective view of one embodiment of the needle assembly component of the present invention including a modified hub.

FIGURE 4 is a perspective view of the shield component of the invention usable with the needle assembly depicted in FIGURE 3.

FIGURE 5 is a perspective view of the embodiment of the invention depicted in FIGURE 3 with the shield depicted in FIGURE 4 positioned over the hub of the needle assembly so as to recess the distal end of the needle.

FIGURE 6 is a side cross-sectional view of an embodiment of the present invention including a catheter unit.

Like reference characters in the various drawings refer to like elements.

### DETAILED DESCRIPTION OF THE INVENTION

The following description is of the best presently contemplated mode of carrying out the invention. This description is made for the purpose of illustrating the general principles of the invention and should not be taken in a limiting sense. The scope of the invention is best determined by reference to the appended claims.

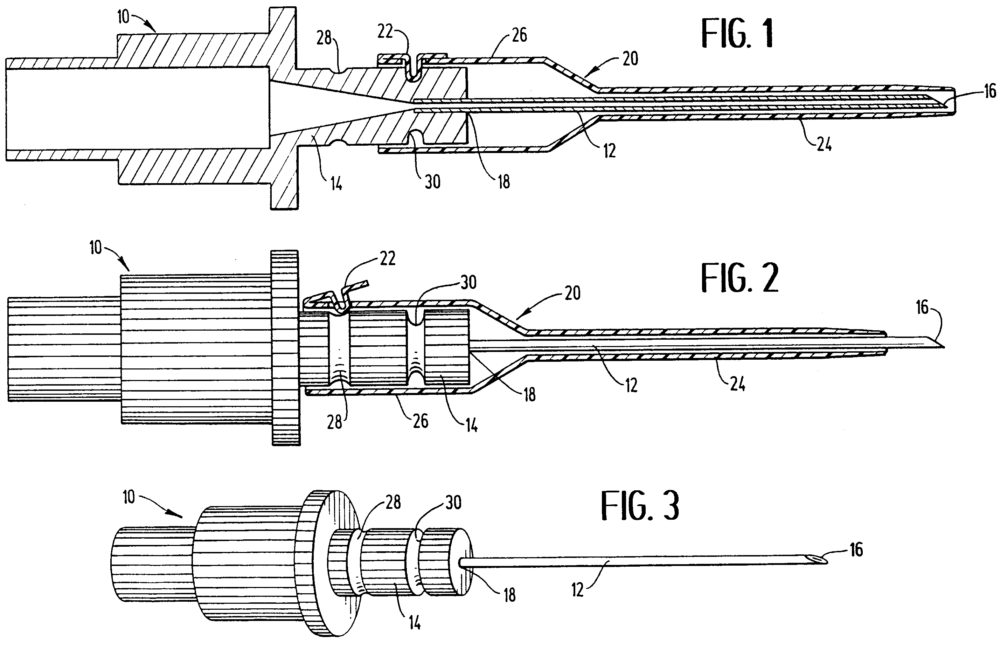

Referring now to the drawings, FIGURES 1-5 show the components of the preferred embodiment of the inventive shield assembly. The shield assembly includes a needle assembly 10 having a hollow needle cannula 12 and a hub 14. The needle cannula 12 is provided with a sharp distal end 16 designed to pierce the skin, subcutaneous tissue and blood vessels of a patient for insertion into the blood vessel. The proximal end 18 of the needle cannula 12 is attached to the hub 14 of the needle assembly 10.

The inventive shield assembly further includes a shield 20 slidably mounted over the needle cannula 12 and the hub 14 of the needle assembly. The shield 20 is provided with a locking member 22 by which the shield 20 is releasably retained on the hub in a first position wherein the distal end 16 of the needle cannula 12 is exposed, or lockingly retained on the hub 14 in a second position wherein the distal end 16 of the needle cannula 12 is recessed within the shield 20. The shield 20 is preferably shaped to include a cannula-covering section 24 having a diameter slightly larger than the diameter of the needle cannula 12 to fit flush thereover and a hub-covering section 26 having a diameter slightly larger than the diameter of the hub 14 of the needle assembly 10 to fit flush over the hub 14. However, other shield designs may be used, e.g., a tube having a uniform diameter slightly larger than the hub 14 of the needle assembly 10. The shield is preferably made of a hard plastic.

In the preferred embodiment, the hub 14 of the needle assembly 10 is provided with a pair of annular grooves (see e.g., FIGURE 3). The first groove 28 is shallower than the second groove 30 which is located closer to the distal end 16 of the needle cannula 12. In this embodiment, the locking member 22 of the shield 20 comprises a flexible clip sized to releasably fit within the first groove 28 and to lockingly seat within the second groove 30. The shield 20 has a length whereby when the flexible clip 22 is retained in the first groove 28 the distal end 16 of the needle cannula 12 is exposed. However, when the shield is pushed forward to allow the flexible clip 22 to seat within the second groove 30, the distal end 16 of shield 12 is covered by the shield. The flexible clip 22 is preferably made of metal and formed as an integral part of the hub covering-portion of the shield.

While each of the FIGURES depicts the flexible clip-grooved hub locking arrangement, other locking arrangements may be utilized. For example, a hub 14 of the needle assembly may be provided with a plurality of indented formation or slots spaced along its length and a hub-covering portion of the shield 20 may include one or more complementary detented formations. Alternatively, the locking arrangement may comprise a twisting or rotary mechanism wherein the hub 14 and shield 20 are provided with complementary grooves or threads.

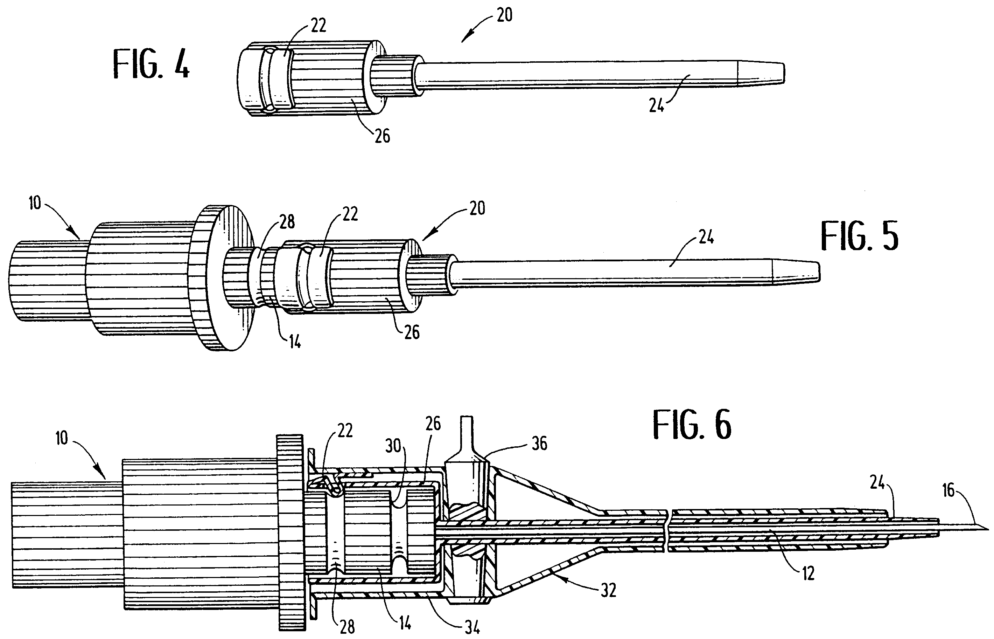

FIGURE 6 shows an embodiment of the present invention wherein the shield assembly includes an intravenous catheter 32 in addition to the needle assembly 10 and shield 20. The catheter 32 of this embodiment includes a hub section 34 and is sized to slidably fit over the shield 20. The catheter 32 preferably has a length slightly shorter than the shield 20. In this embodiment, when the catheter 32 is positioned over shield 20 so that its hub section 34 covers the hub-covering section 26 of the shield 20, the distal end of the cannula-covering section 24 of the shield 20 is exposed (see FIGURE 6).

In the embodiment of the invention depicted in FIGURE 6, the hub-covering section 26 of the shield 20 is modified to permit incorporation of a valve assembly 36 in the hub section 34 of the catheter unit 32. It is advantageous to incorporate a valve 36 into the catheter 32 to prevent the uninhibited flow of blood from the catheter after it has been inserted into a blood vessel and the needle cannula 12 and shield 20 have been withdrawn. Contamination of the area surrounding the insertion site by the patient's blood may thus be prevented. Any valve which permits insertion of the needle assembly 10 and shield 20 and allows repeated opening and closing may be used. Suitable valve assemblies include, for example, simple shuttle cock valves or stop-cock arrangements or more costly plastic membrane-"O"-ring assemblies mounted inside the catheter near its hub section.

The inventive shield assembly including a catheter unit 32 is packaged with the shield 20 in its first position exposing the distal end 16 of the needle cannula 12, and the catheter hub section 34 positioned over the hub-covering section 26 of the shield 20 to expose the distal end of the shield 20. In use, the exposed distal end 16 of the needle cannula 12 pierces the desired blood vessel. As the needle cannula 12 is inserted into the blood vessel, the shield 20 is inserted as well. The shield 20 acts as a dilator by slightly widening the insertion site to permit the catheter's smooth entry into the blood vessel. The catheter 32 may then be advanced over the shield 20 until only its hub section 34 remains external to the insertion site. At this point, the shield 20 is pushed forward to dislodge the locking member 22 from its first position and slide it into its second position covering the distal end 16 of the needle cannula 12. The needle assembly 10 including its fully covered needle cannula 12 is then withdrawn from the blood vessel and set aside or discarded.

The needle assembly 10 of the inventive shield assembly including an intravenous catheter may be attached to a syringe. Upon proper insertion of the needle cannula 12 into the desired blood vessel, blood will flow through the needle assembly 10 into the syringe verifying the blood vessel has been penetrated. Attachment of a syringe to the needle assembly for placement of the catheter furthermore permits immediate withdrawal of a blood sample for analysis.

The inventive shield assembly, as described above, provides a cost-efficient device for substantially eliminating the risk of needle stick injuries by contaminated needles to medical and clean-up personnel. A slight modification of the standard needle hub suffices to incorporate the shield component of the inventive shield assembly into most needle-bearing medical devices. Importantly, incorporation of the inventive shield assembly does not interfere with any of the functions of needles used with hypodermic syringes or catheters and these devices may be handled in accordance with standard techniques, i.e., users need not learn new handling skills to successfully prevent needle stick injuries.

Several embodiments of the present invention have been described. However, it will be understood that various modifications may be made without departing from the spirit and scope of the invention. For example, the various elements of the device may be made of a variety of materials and in different general shapes. Accordingly, it is to be understood that the invention is not to be limited by the specific illustrated embodiments, but only by the scope of the appended claims.

## Claims

1. A shield assembly for shielding the needle of a needle-bearing medical device, comprising:
(a) a needle assembly including a hollow needle cannula for penetrating a body organ and having a sharp distal end and a proximal end, and a hub connecting to the proximal end of the needle cannula;
(b) means for shielding the needle cannula movably mounted over the needle cannula and the hub, and being sized to fit flush over and conform to the needle cannula so as to follow the needle cannula into a body organ upon penetration of the needle cannula into the body organ, the means for shielding being movable from a first position on the hub wherein the sharp distal end of the needle cannula is exposed prior to penetration into a body organ, to a second position on the hub wherein the sharp distal end of the needle cannula is recessed within the means for shielding the needle cannula; and
(c) locking means for retaining the means for shielding in the first or the second position.

2. The shield assembly of claim 1, wherein the locking means releasably retains the means for shielding in the first position and lockingly retains the means for shielding in the second position.

3. The shield assembly of claim 1, wherein the means for shielding is movable from the first position to the second position by a sliding action.

4. The shield assembly of claim 1, wherein the means for shielding is movable from the first position to the second position by a rotary action.

5. The shield assembly of claim 1, wherein the means for shielding is movable from the first position to the second position by a twisting action.

6. The shield assembly of claim 1, wherein the hub of the needle assembly is provided with a plurality of annular grooves and wherein the locking means comprises a flexible clip provided on the means for shielding the needle cannula, the flexible clip being shaped to releasably fit within a first groove and to lockingly fit within a second groove of the plurality of grooves.

7. The shield assembly of claim 6, wherein the first groove of the hub is shallower than the second groove of the hub, the second groove being located closer to the distal end of the needle cannula.

8. The shield assembly of claim 7, wherein the means for shielding the needle cannula comprises a hub-covering portion sized to fit flush over the hub of the needle assembly and wherein the clip is formed integrally with the hub portion of the shield.

9. The shield assembly of claim 1 further comprising an intravenous catheter slidably mounted over the means for shielding the needle cannula.

10. The shield assembly of claim 9, wherein the means for shielding the needle cannula includes a hub-covering portion sized to fit flush over the hub of the needle assembly, and wherein the intravenous catheter includes a hub section sized to fit flush over the hub-covering portion of the means for shielding the needle cannula and a cannula section sized to fit flush over the cannula-covering portion of the means for shielding the needle cannula

11. The shield assembly of claim 10, wherein the hub of the needle assembly is provided with a plurality of annular grooves and wherein the locking means comprises a flexible clip positioned on the hub-covering portion of the means for shielding the needle cannula, the flexible clip being sized to releasably fit within a first groove and to lockingly fit within a second groove of the plurality of grooves.

12. The shield assembly of claim 9, wherein the intravenous catheter includes means for controlling the flow of blood through the catheter upon placement of the catheter into a blood vessel.

13. The shield assembly of claim 12, wherein the means for controlling the flow of blood comprises a valve mounted in the hub section of the intravenous catheter.

14. A method for shielding a needle of a needle-bearing medical device which comprises a needle assembly including a hollow needle cannula for penetrating a body organ having a sharp distal end and a proximal end, and a hub connecting to the proximal end of the needle cannula, the method comprising the steps of:
(a) providing shielding means for shielding the needle cannula, the shielding means being movably mounted over the needle cannula and the hub, and being sized to fit flush over and conform to the needle cannula so as to follow the needle cannula into the body organ, the shielding means being movable between a first position where the sharp distal end of the needle cannula is exposed and a second position where the sharp distal end of the needle is recessed within the shielding means;
(b) penetrating a body organ with the needle cannula and the shielding means, wherein the shielding means is maintained in the first position when the needle cannula and the shielding means penetrate the body organ; and
(c) moving the shielding means to the second position to shield the sharp distal end of the needle cannula within the shielding means after penetrating the body organ with the needle cannula and the shielding means.

15. A method for shielding a needle according to claim 14, further including the step of removing the needle cannula and the shielding means from the body organ, wherein the step of moving the shielding means is performed prior to removing the needle cannula and the shielding means from the body organ.

16. A method for shielding a needle according to claim 14, further including the step of removing the needle cannula and the shielding means from the body organ, wherein the step of moving the shielding means is performed after removing the needle canula and the shielding means from the body organ.
